# FASCICULE DE BREVET EUROPEEN

(11) **EP 1 680 400 B1**
(45) Date de publication et mention de la délivrance du brevet: **06.06.2007**
(21) Numéro de dépôt: 04791555.8
(22) Date de dépôt: 15.10.2004
(51) Int. Cl.: C07D 207/09

(54) **DERIVES DE N-[ PHENYL(PYRROLIDIN-2-YL)METHYL]BENZAMIDE, ET N-[(AZEPAN-2-YL) PHENYLMETHYL]BENZAMIDE, LEUR PREPARATION ET LEUR APPLICATION EN THERAPEUTIQUE**
DERIVATE VON N-[PHENYL(PYRROLIDIN-2-YL)METHYL]BENZAMID UND N-[(AZEPAN-2-YL)PHENYLMETHYL]BENZAMID, VERFAHREN ZU DEREN HERSTELLUNG UND DEREN ANWENDUNG IN THERAPEUTIKA
DERIVATIVES OF N-[ PHENYL(PYRROLIDINE-2-YL)METHYL]BENZAMIDE AND N-[ (AZEPAN-2-YL)PHENYLMETHYL]BENZAMIDE, PREPARATION METHOD THEREOF AND APPLICATION OF SAME IN THERAPEUTICS

(30) Priorité: 17.10.2003 FR 0312143
(43) Date de publication de la demande: 19.07.2006
(73) Titulaire: Sanofi-Aventis, 75013 Paris (FR)
(72) Inventeur: DARGAZANLI, Gihad, F-94230 Cachan (FR); ESTENNE-BOUHTOU, Geneviève, F-94550 Chevilly-Larue (FR); MEDAISKO, Florence, F-94100 Saint-Maur-Des-Fosses (FR); RAKOTOARISOA, Nathalie, F-91160 Longjumeau (FR)
(74) Mandataire: Monain, Patrice
(86) Numéro de dépôt international: PCT/FR2004/002644
(87) Numéro de publication internationale: WO 2005/037785

(56) Documents cités:
- WO-A-99/45011

## Description

La présente invention a pour objet des composés répondant à la formule générale (I) 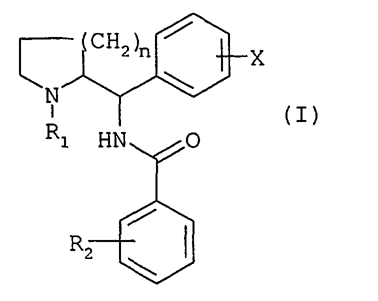 dans laquelle
n représente le nombre 1 ou 3,
R₁ représente soit un atome d'hydrogène, soit un groupe (C₁-C₇)alkyle linéaire ou ramifié éventuellement substitué par un ou plusieurs atomes de fluor, soit un groupe (C₃-C₇) cycloalkyle, soit un groupe (C₃-C₇) cycloalkyl-(C₁-C₃) alkyle, soit un groupe phényl (C₁-C₃) alkyle éventuellement substitué par un ou deux groupes méthoxy, soit un groupe (C₂-C₄)alcényle, soit un groupe (C₂-C₄)alcynyle,
X représente soit un atome d'hydrogène soit un ou plusieurs substituants choisis parmi les atomes d'halogènes et les groupes trifluorométhyle, (C₁-C₆)alkyles et (C₁-C₆)alcoxy. linéaires ou ramifiés,
R₂ représente soit un atome d'hydrogène, soit un ou plusieurs substituants choisis parmi les atomes d'halogènes, et les groupes trifluorométhyle, (C₁-C₆) alkyles, (C₁-C₆) alcoxy, linéaires ou ramifiés, (C₃-C₇)cycloalkyles, phényle, cyano, acétyle, benzoyle, S(C₁-C₆)alkyles, (C₁-C₆)alkylsulfonyles, carboxy et (C₁-C₆)alcoxycarbonyles, soit un groupe de formule générale NR₃R₄, SO₂NR₃R₄ ou CONR₃R₄, dans lesquelles R₃ et R₄ représentent chacun, indépendamment l'un de l'autre, un atome d'hydrogène ou un groupe (C₁-C₆)alkyle linéaire ou ramifié ou (C₃-C₇)cycloalkyle, ou forment, avec l'atome d'azote qui les porte, un cycle pyrrolidine, pipéridine ou morpholine.

Les composés de formule générale (I) peuvent exister sous forme des racémates thréo (1*R*, 2*R* ; 1*S*,2*S*) ou érythro (1*S*, 2*R* ; 1*R*,2*S*) ou sous forme d'énantiomères ; ils peuvent exister à l'état de bases libres ou de sels d'addition à des acides.

Des composés de structure analogue à celle des composés de l'invention sont décrits dans le brevet US-5254569 comme analgésiques, diurétiques, anticonvulsivants, anesthésiques, sédatifs, cérébroprotecteurs, par un mécanisme d'action sur les récepteurs opiacés.

Les composés de l'invention présentent une activité particulière comme inhibiteurs spécifiques des transporteurs de la glycine glyt1 et/ou glyt2.

Les composés de formule générale (I) dans laquelle R₁ est différent d'un atome d'hydrogène peuvent être préparés par un procédé illustré par le schéma 1 qui suit. 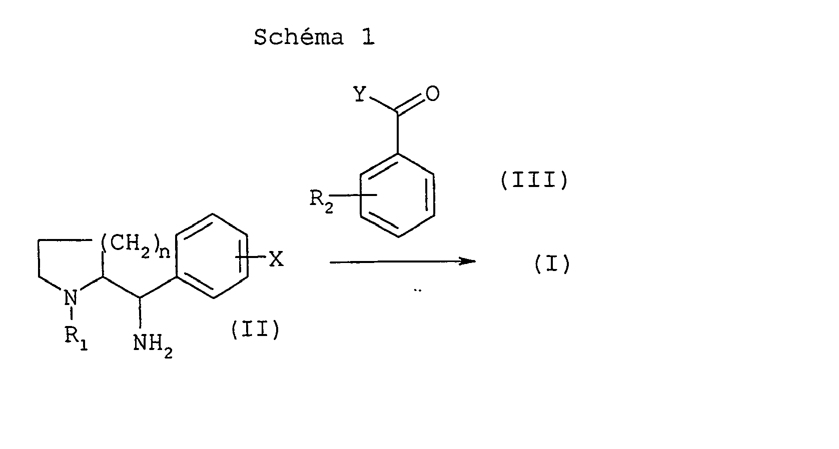 On effectue un couplage d'une diamine de formule générale (II) de configuration relative thréo ou érythro ou en mélange, dans laquelle R₁ et X sont tels que définis ci-dessus (avec R₁ différent d'un atome d'hydrogène) avec un acide activé ou un chlorure d'acide de formule générale (III) dans laquelle Y représente un groupe nucléofuge, tel qu'un un atome d'halogène et R₂ est tel que défini ci-dessus, en utilisant les méthodes connues de l'homme du métier.
On peut obtenir les composés de formule générale (I) érythro ou thréo purs selon toutes méthodes connues de l'homme du métier, par exemple par séparation par chromatographie liquide à haute performance.

Pour n=1 avec R₁ différent de l'atome d'hydrogène et X tels que définis ci-dessus, la diamine de formule générale (II), de configuration relative thréo ou érythro ou en mélange, peut être préparée par le procédé illustré par le schéma 2 voie A.
On peut réduire la cétone IV où P représente Boc en alcool (X) érythro/thréo dont le ratio dépend de la nature de l'hydrure utilisé selon une méthode décrite dans *J.Chem. Soc. Chem. Commun.,* 1986, 412-413. Le groupement protecteur est ensuite éliminé selon une méthode classique , dans un mélange de dichlorométhane et d'acide trifluoroacétique. On obtient ainsi l'amino alcool (XI) sur lequel on procède ensuite à une N-alkylation au moyen d'un dérivé halogéné de formule R₁Z, et d'une base du type potassium carbonate pour donner l'amino alcool fonctionnalisé de formule générale (XII).
Enfin dans les conditions classiques de Mitsunobu, selon une méthode décrite dans *Bull. Soc. Chim. Belg.* **(106)**, 1997, 77-84 en présence d'acide hydrazoïque et de triphénylphosphine, on obtient la diamine de formule générale (II).

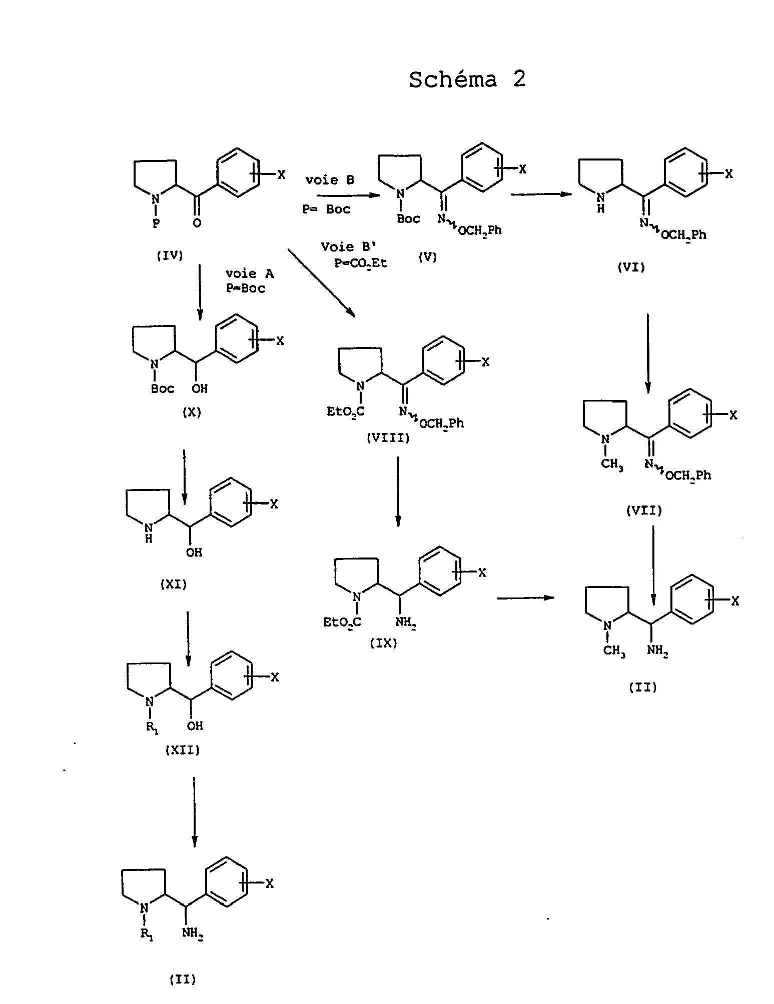

Pour n=1 avec R₁= CH₃ et X tel que défini ci-dessus en peut également obtenir la diamine de formule générale (II), de configuration relative thréo ou érythro ou en mélange, selon les voies B et B' du schéma 2 et selon le schéma 3.

Selon la voie B, on fait réagir la cétone (IV) dans laquelle X est tel que défini ci-dessus, avec le chlorhydrate de la benzylhydroxylamine au reflux de la pyridine pour fournir un mélange d'oxime (V) que l'on déprotège avec l'acide trifluoroacétique pour obtenir l'amine libre (VI).

La méthylation de la pyrrolidine s'effectue classiquement au reflux du formaldéhyde et de l'acide formique pour générer le composé (VII). Enfin l'hydrogénation catalysée par le palladium sur charbon de ce composé, dans un solvant alcoolique en présence d'acide chlorhydrique aqueux conduit à la diamine de formule générale (II).

Selon la voie B', on peut faire réagir la cétone de formule générale (IV) où P représente CO₂Et et X est tel que défini ci-dessus, avec le chlorhydrate de la benzylhydroxylamine au reflux de l'éthanol pour fournir un mélange d'oximes (VIII) sur lesquelles on pratique une hydrogénation catalysée par le palladium sur charbon dans un solvant alcoolique en présence d'acide chlorhydrique aqueux pour fournir le carbamate (IX). La réduction du carbamate de formule générale (IX) par l'hydrure double d'aluminium et de lithium au reflux d'un solvant tel que l'éther conduit à la diamine de formule générale (II).

Selon le schéma 3, l'amino alcool (XIII) est transformé en azoture (XIV) dans les conditions classiques de Mitsunobu, selon une méthode décrite dans *J. Org. Chem.,* **(64)**, 1999, 6106-6111. La réduction du carbamate azoture (XIV) par l'hydrure double d'aluminium et de lithium au reflux d'un solvant tel que le tétrahydrofuranne conduit à un mélange de diamines de formule générale (II).

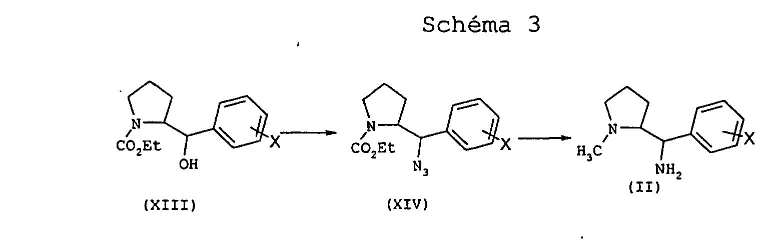

La diamine de formule générale (II) de configuration relative thréo ou érythro dans laquelle R₁ est différent d'un atome d'hydrogène et n = 3 peut être préparée par un procédé illustré par le schéma 4 qui suit.

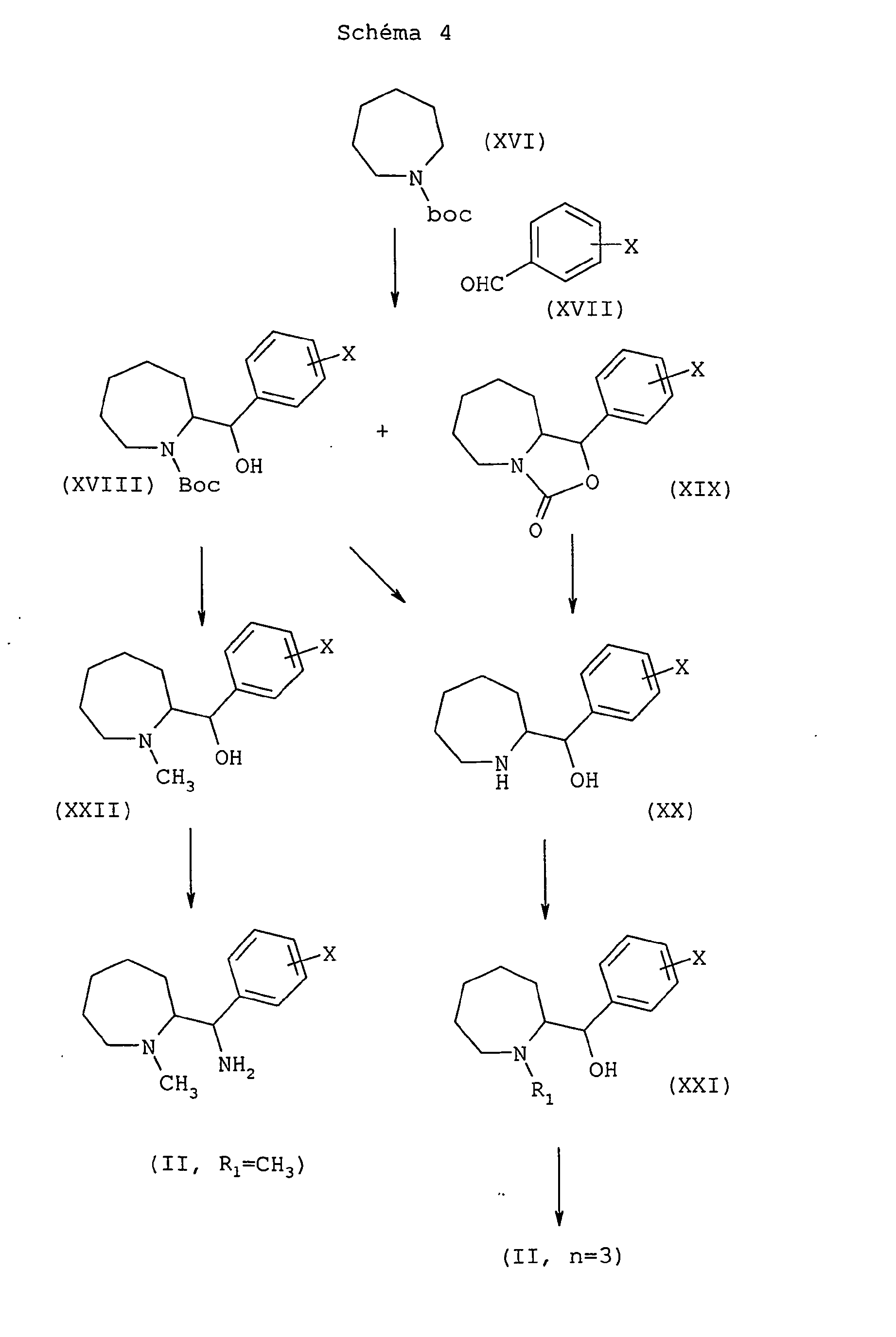

On réalise l'alpha-lithiation de l'azépane de formule générale (XVI) dans laquelle Boc représente un groupe 1,1-diméthyléthoxycarbonyle, par le *sec*-butyllithium en présence de TMEDA (*N,N,N',N*'-tétraméthyléthylènediamine) dans un solvant éthéré tel que l'éther diéthylique à -78°C, pour faire réagir la lithioamine formée *in situ* sur le benzaldéhyde de formule générale (XVII) selon une méthode décrite dans *J. Org. Chem.,* **(58)**, 5, 1993, 1109-1117.
On obtient ainsi un mélange d'alcool de formule générale (XVIII) de configuration érythro et de carbamate cyclique de formule générale (XIX) de configuration thréo Le carbamate de formule générale (XVIII) de configuration érythro peut ensuite être réduit en *N*-méthylaminoalcool érythro de formule générale (XXII) par action d'un hydrure mixte tel que l'hydrure double d'aluminium et de lithium, dans un solvant éthéré tel que le tétrahydrofurane, entre la température ambiante et la température de reflux.
On transforme ensuite l'alcool érythro de formule générale (XXII) en intermédiaire érythro de formule générale (II) où R₁ représente un groupe méthyle, en deux étapes : on transforme d'abord la fonction alcool en groupe nucléofuge, par exemple un groupe méthanesulfonate, par action du chlorure de mésyle, dans un solvant chloré tel que le dichlorométhane, et en présence d'une base telle que la triéthylamine, entre 0°C et la température ambiante, puis on fait réagir le groupe nucléofuge avec de l'ammoniac liquéfié à -50°C, dans un alcool tel que l'éthanol, dans un milieu clos tel qu'un autoclave, entre -50°C et la température ambiante.
On peut également déprotéger le carbamate de formule générale (XVIII) de configuration érythro au moyen d'une base forte telle que la potasse aqueuse, dans un alcool tel que le méthanol pour obtenir 1' aminoalcool correspondant de formule générale (XX). Dans les mêmes conditions d'hydrolyse, le carbamate cyclique thréo de formule générale (XIX) conduit à l'aminoalcool thréo de formule générale (XX).
On procède ensuite à une *N*-alkylation au moyen d'un dérivé halogéné de formule R₁Z, dans laquelle R₁ est tel que défini ci-dessus, mais différent d'un atome d'hydrogène, et Z représente un atome d'halogène, en présence d'une base telle que le carbonate de potassium, dans un solvant polaire tel que le *N,N*-diméthylformamide, entre la température ambiante et 100°C pour conduire au dérivé alkylé de formule générale (XXI). On traite ensuite ce dernier comme décrit à propos de l'alcool de formule générale (XXII).

Les composés de formule générale (I) dans laquelle R₁ représente un atome d'hydrogène peuvent être préparés à partir d'un composé de générale (I) dans laquelle R₁ représente soit un groupe phénylméthyle éventuellement substitué et à déprotéger l'azote du cycle pipéridine, par exemple par un agent oxydant ou par un acide de Lewis tel que le tribromure de bore, ou par hydrogénolyse soit un groupe alcényle, de préférence allyle, suivie d'une déprotection par un complexe du Pd° pour obtenir un composé de formule générale (I) dans laquelle R₁ représente un atome d'hydrogène

Par ailleurs les composés chiraux de formule générale (I) peuvent être également obtenus soit par séparation des composés racémiques par chromatographie liquide à haute performance (CLHP) sur colonne chirale, soit à partir de l'amine chirale obtenue soit par dédoublement de l'amine racémique de formule générale (II) par utilisation d'un acide chiral, tel que l'acide tartrique, l'acide camphorsulfonique, l'acide dibenzoyltartrique, la N-acétylleucine, par la recristallisation fractionnée et préférentielle d'un sel diastéréoisomérique dans un solvant de type alcool, soit par synthèse chirale selon la voie B' ou A à partir de la cétone de formule générale (IV) chirale du schéma 2, soit encore à partir de l'alcool chiral de formule générale (XIII) du schéma 3.

La cétone de formule générale (IV) racémique peut être préparée selon une méthode décrite dans *Tetrahedron Lett.,* **(38)** (5) ,1997, 783-786 ; *Tetrahedron* **,(59),** 2003,1083-1094. En série chirale la cétone de formule générale (IV) ou les alcools chiraux de formules générales (X) ou (XIII) peuvent être préparés selon une méthode décrite dans la demande internationale WO03004468 et dans *J.Chem.Soc. Perkin Trans I,* 1987, 1465-1471. La perhydroazépine de formule générale (XVI) peut être préparée selon une méthode décrite dans *J. Org. Chem.,* **(58)**,5, 1993, 1109-1117.

Les exemples qui vont suivre illustrent la préparation de quelques composés de l'invention. Les microanalyses élémentaires, et les spectres I.R. et R.M.N. et la CLHP sur colonne chirale confirment les structures et les puretés énantiomériques des composés obtenus.
Les numéros indiqués entre parenthèses dans les titres des exemples correspondent à ceux de la 1ère colonne du tableau donné plus loin.
Dans les noms des composés, le tiret "-" fait partie du mot, et le tiret "-" ne sert que pour la coupure en fin de ligne ; il est à supprimer en l'absence de coupure, et ne doit être remplacé ni par un tiret normal ni par un espace.

### Exemple 1 (Composé N°.1).

### Chlorhydrate de thréo-2-chloro-N-[(1-méthylpyrrolidin-2-yl)phénylméthyl]-3-trifluorométhylbenzamide 1:1.

### 1.1. 2-[[(benzyloxy)imino](phényl)méthyl]pyrrolidine-1-carboxylate de tert-butyle .

Dans un ballon de 1000 ml, muni d'une agitation magnétique, on introduit 8,8 g (31,36 mmoles) de 2-benzoylpyrrolidine-1-carboxylate de *tert*-butyle et 5,6 g (35,15 mmoles) de chlorhydrate de benzylhydroxylamine en solution dans 100 ml d'éthanol absolu et 35 ml de soude 1M et on chauffe au reflux pendant 16 h.

Après évaporation à sec du milieu réactionnel sous pression réduite, on dilue le résidu avec de l'eau et du dichlorométhane, on sépare la phase aqueuse, et on l'extrait avec du dichlorométhane. Après lavage des phases organiques réunies, séchage sur sulfate de sodium et évaporation du solvant sous pression réduite, on purifie le résidu par chromatographie sur colonne de gel de silice en éluant avec un mélange d'acétate d'éthyle et de cyclohexane.
On obtient ainsi 8 g de produit sous forme d'huile.

### 1.2. Phényl(pyrrolidin-2-yl)méthanone O-benzyloxime

Dans un ballon de 500 ml, muni d'une agitation magnétique, on introduit 8g (20 mmoles) 2-[[(benzyloxy)imino] (phényl)-méthyl]pyrrolidine-1-carboxylate de *tert*-butyle en solution dans 400 ml d'un mélange d'acide trifluoroacétique 30% dans le dichlorométhane et on agite 4 h à température ambiante. Après évaporation à sec du milieu réactionnel sous pression réduite, on dilue le résidu avec de l'ammoniaque et du dichlorométhane, on sépare la phase aqueuse, et on l'extrait avec du dichlorométhane. Après lavage des phases organiques réunies, séchage sur sulfate de sodium et évaporation du solvant sous pression réduite, on purifie le résidu par chromatographie sur colonne de gel de silice en éluant avec un mélange dichlorométhane et de méthanol.
On obtient 4 g de produit.

1.3(1-méthylpyrrolidin-2-yl)(phényl)méthanone *O*-benzyloxime. Dans un ballon de 50 ml , muni d'une agitation magnétique, on introduit 1,2 g (4,28 mmoles) de phényl(pyrrolidin-2-yl)méthanone *○*-benzyloxime dans 4 ml d'un mélange (1/1) d'acide formique et de formaldéhyde aqueux à 37% et on chauffe au reflux pendant 16 h.
Après évaporation à sec du milieu réactionnel sous pression réduite, on dilue le résidu avec de l'ammoniaque et du dichlorométhane, on sépare la phase aqueuse, et on l'extrait avec du dichlorométhane. Après lavage des phases organiques réunies, séchage sur sulfate de sodium et évaporation du solvant sous pression réduite, on purifie le résidu par chromatographie sur colonne de gel de silice en éluant avec un mélange de dichlorométhane et de méthanol.
On obtient 1,05 g de produit.

### 1.4 [(1-Méthylpyrrolidin-2-yl)phényl)méthyl]amine.

Dans une fiole de Parr sous atmosphère d'azote on place 1,05 g (3,56 mmoles) de (1-méthylpyrrolidin-2-yl)(phényl)-méthanone *O*-benzyloxime en solution dans un mélange de 20 ml d'éthanol et de 10 ml d'acide chlorhydrique 1N en présence d'une pointe de spatule de palladium sur charbon à 10%. On place les réactifs sous atmosphère d'hydrogène et on les agite pendant 8 h.
Après filtration du catalyseur et évaporation du filtrat sous pression réduite, on dilue le résidu avec de l'ammoniaque et du dichlorométhane, on sépare la phase aqueuse, et on l'extrait avec du dichlorométhane. Après lavage des phases organiques réunies, séchage sur sulfate de sodium et évaporation du solvant sous pression réduite,on obtient ainsi 0,54 g de produit sous forme d'une huile que l'on utilise brute dans l'étape suivante.

### 1.5 Chlorhydrate de thréo-2-chloro-N-[(1-méthylpyrrolidin-2-yl)phénylméthyl]-3-trifluorométhylbenzamide 1:1.

Dans ballon de 100 ml , sous atmosphère d'azote on place 0,54 g (2,84 mmoles) de [(1-méthylpyrrolidin-2-yl)phényl)méthyl]amine et 0,41 g de carbonate de potassium en solution dans 7 ml de dichlorométhane à 0°C. On ajoute une solution de 0,72 g (2,97 mmoles) de chlorure d'acide 2-chloro-3-trifluorométhylbenzoïque en solution dans 3 ml de dichlorométhane et on laisse 16 h à température ambiante.
On dilue le mélange réactionnel avec de l'eau et du dichlorométhane, on sépare la phase aqueuse, et on l'extrait avec du dichlorométhane. Après lavage des phases organiques réunies, séchage sur sulfate de sodium et évaporation du solvant sous pression réduite, on purifie le résidu par chromatographie sur colonne de gel de silice en éluant avec un mélange de dichlorométhane et de méthanol. On isole ainsi 110 mg de thréo-2-chloro-*N*-[(1-méthylpyrrolidin-2-yl)phényl méthyl]-3-trifluorométhylbenzamide.
On dissout ce dernier dans quelques ml de propan-2-ol, on ajoute 6 ml d'une solution 0,1N d'acide chlorhydrique dans le propan-2-ol, et on concentre le mélange sous pression réduite afin de réduire le volume du solvant. Après trituration on isole finalement 0,10 g de chlorhydrate sous forme d'un solide.
Point de fusion : 96-110°C.

### Exemple 2 (Composé N°2).

### Chlorhydrate de thréo-4-amino-3,5-dichloro-N-[(1-méthylpyrrolidin-2-yl)phénylméthyl]benzamide 1:1.

Dans ballon de 100 ml muni d'une agitation magnétique on introduit 0,975 g (4,73 mmoles) d'acide 4-amino-3,5-dichlorobenzoïque, 0,639 g (4,73 mmoles) d'hydroxybenzotriazole 0,906 g (4,73 mmoles)de chlorhydrate de 1-[3-(diméthylamino)propyl]-3-éthylcarbodiimide en solution dans 50 ml de dichlorométhane. On laisse 30 min à température ambiante et on ajoute 0,9 g (4,73 mmoles) de [(1-méthylpyrrolidin-2-yl)phényl)méthyl]amine en solution dans 20 ml de dichlorométhane et on laisse à température ambiante pendant la nuit.
Après hydrolyse à l'eau et dilution au dichlorométhane on sépare la phase aqueuse, et on l'extrait avec du dichlorométhane. Après lavage des phases organiques réunies, séchage sur sulfate de sodium et évaporation du solvant sous pression réduite, on purifie le résidu par chromatographie sur colonne de gel de silice en éluant avec un mélange de dichlorométhane et de méthanol.
On obtient 0,19 g de produit huileux.
On dissout ce dernier dans quelques ml de propan-2-ol, on ajoute 20 ml d'une solution 0,1N d'acide chlorhydrique dans le propan-2-ol, et on concentre le mélange sous pression réduite afin de réduire le volume du solvant. Après trituration on isole finalement 0,19 g de chlorhydrate sous forme d'un solide.
Point de fusion : 155-162°C.

### Exemple 3 (Composé N°3).

### Thréo-N-[(1-allylpyrrolidin-2-yl)phényl méthyl]-2-chloro-3-trifluorométhylbenzamide 1:1.

### 3.1. Erythro-2-[hydroxy(phénylméthyl]pyrrolidine-1-carboxylate de tert-butyle.

Dans un tricol de 250 ml muni d'une agitation magnétique, sous atmosphère d'azote, on place 3 g (10,89 mmoles) de 2-benzoylpyrrolidine-1-carboxylate de *tert*-butyle en solution dans 110 ml de tétrahydrofurane à -70°C. On ajoute goutte à goutte 29 ml (43,58 mmoles) d'une solution d'hydrure de diisobutylaluminium 1,5M dans le toluène. On laisse 2 heures à -70°C et on laisse remonter à -20°C. On hydrolyse alors précautionneusement avec 50 ml de méthanol. Après évaporation du mélange réactionnel sous pression réduite, on dilue le résidu avec de l'acide chlorhydrique 1N et du dichlorométhane, on sépare la phase aqueuse, et on l'extrait avec du dichlorométhane. Après lavage des phases organiques réunies, séchage sur sulfate de sodium et évaporation du solvant sous pression réduite, on obtient 2,8 g d'un mélange contenant majoritairement le diastéréo-isomère érythro-2-[hydroxy(phénylméthyl]pyrrolidine-1-carboxylate de *tert*-butyle que l'on utilise brut dans l'étape suivante.

### 3.2. Trifluoroacétate de érythro-phényl(pyrrolidin-2-yl)méthanol.

Dans un ballon de 250 ml, muni d'une agitation magnétique, on place 5 g (21,99 mmoles) d'érythro-2-[hydroxy(phénylméthyl]pyrrolidine-1-carboxylate de *tert-*butyle en solution dans un mélange de 75 ml de dichlorométhane et de 30 ml d'acide trifluoroacétique et on agite le mélange. On laisse à température ambiante pendant 2 h.
On évapore le mélange réactionnel sous pression réduite.
On obtient ainsi 5 g d'un mélange contenant le trifluroacétate de l'érythro-phényl(pyrrolidin-2-yl)méthanol que l'on utilise brut dans l'étape suivante.

### 3.3 Erythro-(1-allylpyrrolidinyl-2-yl)phényl)méthanol.

Dans un ballon de 250 ml, muni d'une agitation magnétique, on.place 5 g (17,16 mmoles) de trifluoroacétate de érythrophényl(pyrrolidin-2-yl)méthanol, 5,9 g (43 mmoles) de carbonate de potassium et 1,8 ml (20,6 mmoles) de bromure d'allyle en solution dans 50 ml d'acétonitrile, et on agite à température ambiante pendant 16 h.
Après évaporation à sec du milieu réactionnel sous pression réduite, on dilue le résidu avec de l'ammoniaque et du dichlorométhane, on sépare la phase aqueuse, et on l'extrait avec du dichlorométhane. Après lavage des phases organiques réunies, séchage sur sulfate de sodium et évaporation du solvant sous pression réduite, on purifie le résidu par chromatographie sur colonne de gel de silice en éluant avec un mélange de dichlorométhane et de méthanol.
On obtient ainsi 1,1g d'un mélange contenant de l'érythro-(1-allylpyrrolidinyl-2-yl)phényl)méthanol.

### 3.4. Erythro- [(1-allylpyrrolidinyl-2-yl)phényl)méthyl] amine. Dans un tricol de 100 ml muni d'une agitation magnétique sous atmosphère d'azote on introduit 1,1 g (5,06 mmoles) d'érythro-1-(allylpyrrolidinyl-2-yl)phényl)méthanol et 1,6 g (6,07 mmole)de triphénylphosphine en solution dans 15 ml de tétrahydrofurane. On ajoute 6 ml d'une solution 1M dans le benzène (6 mmoles) d'acide hydrazoïque. A cette solution on ajoute, goutte à goutte, une solution de 1,09 ml (0,56 mmole) de diisopropylcarbodiimide dans 10 ml de tétrahydrofurane. On chauffe à 40°C pendant 16h puis on ajoute 1,3 g(5,06 mmoles) de triphénylphosphine, on agite pendant 30 min puis on ajoute 0,6 ml d'eau et on poursuit l'agitation pendant 6 h.

On hydrolyse avec de l'acide chlorhydrique 1N et on dilue avec du chloroforme. On basifie la phase aqueuse avec de l'ammoniaque et on l'extrait plusieurs fois avec du chloroforme. Après lavage des phases organiques réunies, séchage sur sulfate de sodium et évaporation du solvant sous pression réduite. On obtient 1 g d'une huile orangée contenant de la thréo-[(1-allylpyrrolidinyl-2-yl)phényl)méthyl]amine que l'on utilise brute dans l'étape suivante

### 3.5 Thréo-N-[(1-allylpyrrolidin-2-yl)phényl méthyl]-2-chloro-3-trifluorométhylbenzamide

Selon le mode opératoire décrit dans l'exemple 1.5, en partant de 1 g (4,62 mmoles) de thréo [(1-allylpyrrolidin-2-yl)phényl)méthyl]amine, 1,13 g (4,62 mmoles) de chlorure d'acide 2-chloro-3-trifluorométhylbenzoïque et 0,64 g (4,62 mmoles) de carbonate de potassium, on obtient 20 mg d'une huile qui cristallise
Point de fusion : 117-123°C.

### Exemple 4 (Composé N°4).

### Chlorhydrate de 3-(aminosulfonyl)-4-chloro-N-[(S)-[(2S)-1-méthylpyrrolidin-2-yl](phényl)méthyl]benzamide 1:1.

### 4.1. 2-[(Benzyloxy)imino]phénylméthylpyrrolidine-1-carboxylate d'éthyle.

Dans un ballon de 100 ml muni d'une agitation magnétique, on introduit 1,36 g (5,5 mmoles) de 2-benzoylpyrrolidine-1-carboxylate d'éthyle en solution de 30 ml d'éthanol et on ajoute 1,75 g (10,96 mmoles) de chlorhydrate de benzylhydroxylamine et on chauffe le mélange au reflux pendant 12 h. Après évaporation du solvant sous pression réduite, on reprend le résidu à l'acétate d'éthyle et on lave la phase organique avec une solution saturée en chlorure de sodium, on la sèche sur sulfate de sodium et on l'évapore sous pression réduite. On obtient 1,95 g d'une huile jaune que l'on purifie par chromatographie sur colonne de gel de silice en éluant avec un mélange d'acétate d'éthyle et de cyclohexane.
On obtient 1,56 g de produit.

### 4.2 (S)-2-[(S)-amino(phényl)méthyl]pyrrolidine-1-carboxylate d'éthyle et [phényl(-pyrrolidin-2-yl)méthyl]carbamate d'éthyle.

Dans une fiole de Parr de 250 ml, on introduit 1,56 g (4,43 mmoles) de [(benzyloxy)imino]phénylméthylpyrrolidine-1-carboxylate d'éthyle dans 40 ml d'éthanol et 8 ml d'acide chlorhydrique 1N, on ajoute 0,15 g de palladium sur charbon à 10% et on place le mélange sous atmosphère d'hydrogène pendant 7 h.
Après filtration du catalyseur et évaporation du filtrat sous pression réduite, on dilue le résidu avec de l'ammoniaque et du dichlorométhane, on sépare la phase aqueuse, et on l'extrait avec du dichlorométhane.. Après lavage des phases organiques réunies, séchage sur sulfate de sodium et évaporation du solvant sous pression réduite, on obtient 1 g d'un mélange comprenant les (*S*)-2-[(*S*)-amino(phényl)méthyl]pyrrolidine-1-carboxylate d'éthyle et le [phênyl(-pyrrolidin-2-yl)méthyl]carbamate d'éthyle, que l'on utilise brut dans l'étape suivante.

### 4.3 [(S)-[(2S)-(1-méthylpyrrolidin-2-yl)]phényl)-méthyl]amine.

Dans un ballon de 100 ml muni d'une agitation magnétique, sous atmosphère d'azote, on introduit 1 g (4 mmoles) du mélange comprenant les (*S*)-2-[(*S*)-amino(phényl)méthyl]-pyrrolidine-1-carboxylate d'éthyle et [phényl(-pyrrolidin-2-yl)méthyl]carbamate d'éthyle en solution dans 20 ml d'éther anhydre à 0°C. On ajoute 0,8 g (21 mmoles) d'hydrure double d'aluminium et de lithium par portions et on chauffe au reflux pendant 5 h.
Après refroidissement, on traite successivement par 0,8 ml d'eau, 0,8 ml de soude à 15% et 2,4 ml d'eau.
Après filtration sur Célite®, on concentre le filtrat sous pression réduite. On purifie le résidu obtenu (0,7g) par chromatographie sur colonne de gel de silice en éluant avec un mélange de dichlorométane de méthanol et d'ammoniaque.
On obtient 0,12 g de produit sous forme d'une huile jaune.

### 4.4 Chlorhydrate de 3-(aminosulfonyl)-4-chloro-N-[(S)-[(2S)-1-méthylpyrrolidin-2-yl](phényl)méthyl]benzamide 1:1.

Selon le mode opératoire décrit dans l'exemple 2, en partant de 0,12 g (0,63 mmole)de [(*S*)-[(2*S*)-(1-méthylpyrrolidin-2-yl)]phényl)méthyl]amine, 0,12 g (0,63 mmole)de chlorhydrate de 1-[3-(diméthylamino)propyl]-3-éthylcarbodiimide, 0,085 g (0, 63 mmole) d'hydroxybenzotriazole et 0,14 g (0,63 mmole) d'acide 4-chloro-3-sulfonylbenzoïque, on obtient, après traitement et purification par chromatographie sur gel de silice avec un gradient de dichlorométhane et de méthanol, 0,12 g de 3-(aminosulfonyl)-4-chloro-*N*-[(S)-[(2*S*)-1-méthylpyrrolidin-2-yl](phényl)méthyl]benzamide.
On dissout ce dernier dans quelques ml de propan-2-ol, on ajoute 20 ml d'une solution 0,1N d'acide chlorhydrique dans le propan-2-ol, et on concentre le mélange sous pression réduite afin de réduire le volume du solvant. Après trituration on isole finalement 0,09 g de chlorhydrate sous forme d'un solide blanc.
Point de fusion : 165-170°C.

### Exemple 5 (Composé N°5)

### Chlorhydrate de érythro-4-amino-3-chloro-N-[1-méthylpyrrolidinyl-2-yl] (phényl)méthyl]-5-(trifluorométhyl) benzamide 1:1

### 5.1 Erythro-[azido(phényl)méthyl]pyrrolidine-1-carboxylate d'éthyle.

Dans un ballon de 500 ml muni d'une agitation magnétique et sous atmosphère d'argon, on place 2,9 g (11,6 mmoles) de thréo-[hydroxy(phényl)méthyl]pyrrolidine-1-carboxylate d'éthyle en solution dans 150 ml de tétrahydrofurane à 0°C. On ajoute 4,57 g (17,4 mmoles)de triphénylphosphine et 35 mmoles d'une solution d'acide hydrazoïque dans le toluène. On ajoute goutte à goutte 2,74 ml (17,4 mmoles) d'azidodicarboxylate d'éthyle et on laisse agiter pendant 24 h.
On ajoute de la soude 1N et on reprend le mélange repris avec de l'acétate d'éthyle. On sèche la phase organique sur sulfate de sodium et on l'évapore sous pression réduite. On obtient 10 g d'un résidu que l'on purifie par chromatographie sur gel de silice avec un gradient de cyclohexane et d'acétate d'éthyle. On obtient ainsi 1,17 g d'érythro-[azido(phényl)méthyl]pyrrolidine-1-carboxylate d'éthyle.

### 5.2 Erythro-[(1-méthylpyrrolidin-2-yl)phényl)méthyl]amine Dans tricol de 100 ml muni d'une agitation magnétique sous argon, on place 0,8 g (21,32 mmoles) d'hydrure double de lithium et d'aluminium dans 25 ml de tétrahydrofurane et on ajoute une solution de 1,17 g (4,26 mmoles) d'érythro [azido(phényl)méthyl]pyrrolidine-1-carboxylate d'éthyle dans 10 ml de tétrahydrofurane et on chauffe le mélange à 70°C pendant 2 h.

Après refroidissement on traite successivement avec 0,8 ml d'eau, 0,8 ml de soude à 15% et 2,4 ml d'eau.
Après filtration sur Célite®, on évapore le filtrat sous pression réduite et on purifie le résidu par chromatographie sur gel de silice avec un mélange de dichlorométhane, de méthanol et d'ammoniaque. On obtient ainsi 0,16 g d'érythro-[(1-méthylpyrrolidin-2-yl)phényl)méthyl]amine et 0,15 g de [méthylphényl(pyrrolidinyl-2-yl)méthyl]amine.

### 5.3 Chlorhydrate de érythro-4-amino-3-chloro-N-[1-méthylpyrrolidinyl-2-yl](phényl)méthyl]-5-(trifluorométhyl)benzamide 1:1.

Selon le mode opératoire décrit dans l'exemple 2, en partant de 0,073 g (0,38 mmole)de érythro (1-méthylpyrrolidin-2-yl)]phényl)méthyl]amine, 0, 074 g (0,38 mmole) de chlorhydrate de 1-[3-(diméthylamino)propyl]-3-éthylcarbodiimide, 0,052 g (0,38 mmole) d'hydroxybenzotriazole et 0,092 g (0,63 mmole) d'acide 4-amino-3-chloro-5-trifluorométhylbenzoïque, on obtient après traitement et purification par chromatographie sur gel de silice avec un gradient de dichlorométhane et de méthanol, 0,089 g de érythro-4-amino-3-chloro-N-[1-méthylpyrrolidinyl-2-yl](phényl)méthyl]-5-(trifluorométhyl)benzamide.
On dissout ce dernier dans quelques ml de propan-2-ol, on ajoute 20 ml d'une solution 0,1N d'acide chlorhydrique dans le propan-2-ol, et on concentre le mélange sous pression réduite afin de réduire le volume du solvant. Après trituration on isole finalement 0,07 g de chlorhydrate sous forme d'un solide blanc.
Point de fusion : 130-140°C.

### Exemple 6 (Composé N°6).

### Chlorhydrate de 3-(aminosulfonyl)-4-chloro-N-[(R)-[(2S)-1-méthylpyrrolidin-2-yl] (phényl)méthyl]benzamide 1:1.

En utilisant la méthode de synthèse de l'exemple 5 en partant de l'amino alcool thréo chiral (2*S*)-2-[2-(*S*)-hydroxy(phényl)méthyl]pyrrolidine-1-carboxylate d'éthyle, on obtient 0,12 g de chlorhydrate de 3-(aminosulfonyl)-4-chloro-N-[(R)-[(2S)-1-méthylpyrrolidin-2-yl](phényl)méthyl]benzamide 1:1.
Point de fusion : 190-192°C.

### Exemple 7 (Composé N°7).

### Chlorhydrate d'érythro-2-chloro-N-[(R)-[(2S)-1-méthylazépan-2-yl] (phényl)méthyl]-3-(trifluorométhyl)benzamide 1:1

### 7.1 2-[Hydroxy(phényl)méthyl]azépane-1-carboxylate de tert-butyle.

Dans un tricol de 250 ml muni d'une agitation magnétique sous atmosphère d'argon, on place 5 g (25,09 mmoles) d'azépane-1-carboxylate de *tert*-butyle et 3,8 ml (25,09 mmoles) de tétraméthylenediamine en solution dans 30 ml d'éther anhydre à -75 °C. On ajoute goutte à goutte 21 ml (27,60 mmoles)de *sec*-butyllithium 1,3M dans le cyclohexane. On laisse remonter la température jusqu'à -50°C en 3 h (solution A).
Dans un ballon de 250 ml , muni d'une agitation magnétique sous atmosphère d'argon, on place 3,8 ml (37,63 mmoles) de benzaldéhyde dans 10 ml d'éther anhydre (solution B).
On refroidit les 2 solutions à -75°C et on introduit la soution A dans la solution B en contrôlant la température. Après la fin de l'addition, on laisse remonter à température ambiante et on laisse agiter une nuit.
Après hydrolyse avec une solution saturée en chlorure d'ammonium, on sépare la phase aqueuse, et on l'extrait avec de l'acétate d'éthyle. Après lavage des phases organiques réunies, séchage sur sulfate de sodium et évaporation du solvant sous pression réduite, on purifie Le résidu (10 g) par chromatographie sur colonne de gel de silice en éluant avec un mélange d'acétate d'éthyle et de cyclohexane.
On obtient ainsi 2g de 2-[hydroxy(phényl)méthyl]azépane-1-carboxylate de *tert*-butyle.

### 7.2 (1-Méthylazépan-2-yl)phényl)méthanol.

Dans un bicol de 100 ml sous atmosphère d'azote, muni d'une agitation magnétique et surmonté d'un réfrigérant, on place en suspension dans 10 ml de tétrahydrofurane 1,2 g (32,74 mmoles) d'hydrure double de lithium et d'aluminium.
On ajoute goutte à goutte une solution de 2 g (6,55 mmoles) de 2-[hydroxy(phényl)méthyl]azépane-1-carboxylate de *tert-*butyle dans 10 ml de tétrahydrofurane et au chauffe au reflux pendant 5 h.
Après refroidissement , on ajoute 5,5 ml d'une solution 0,1M de tartrate double de potassium et de sodium et on agite une nuit à température ambiante.
Après filtration de l'insoluble sous pression réduite et rinçage au tétrahydrofurane, on concentre le filtrat sous pression réduite. On obtient 1,36 g d'une huile que l'on purifie par chromatographie sur colonne de gel de silice en éluant avec un mélange de dichlorométhane, de méthanol et d'ammoniaque.
On obtient 0,95 g de (1-méthylazépan-2-yl)phényl)méthanol.

### 7.3 [(1-Méthylazépan-2-yl)phényl)méthyl]amine.

Dans un ballon de 100 ml sous atmosphère d'azote muni d'une agitation magnétique on place 0,95 g (4,33 mmoles) de (1-méthylazépan-2-yl)phényl)méthanol, 0,6 ml (4,33 mmoles) de triéthylamine en solution dans 20 ml de dichlorométhane à 0°C. On ajoute 0,34 ml de chlorure de mésyle et on agite le mélange à température ambiante pendant 3 h.
Après évaporation des solvants sous pression réduite on reprend le résidu dans 20 ml d'éthanol et on l'ajoute à une solution d'ammoniac liquéfié dans un autoclave refroidi à -50°C. On ferme l'autoclave et on laisse agiter à température ambiante pendant 48 h.
On dilue le mélange réactionnel avec de l'eau et du dichlorométhane. On extrait la phase aqueuse 3 fois au dichlorométhane. Après lavage des phases organiques réunies, séchage sur sulfate de sodium et évaporation du solvant sous pression réduite on obtient 1,7 g de [(1-méthylazépan-2-yl)phényl)méthyl]amine sous forme d'huile que l'on utilise brute dans l'étape suivante.

### 7.4 Chlorhydrate d'érythro-2-chloro-N-(1-méthylazépan-2-yl)(phényl)méthyl]-3-(trifluorométhyl)benzamide 1:1.

Selon le mode opératoire décrit dans l'exemple 2, en partant de 1,7 g (7,79 mmoles)de [(1-méthylazépan-2-yl)phényl)-méthyl]amine, 1,49 g (7,79 mmoles)de chlorhydrate de 1-[3-(diméthylamino)propyl]-3-éthylcarbodiimide, 1,05g (7,79 mmoles) d'hydroxybenzotriazole et 1,74 g (7,79 mmoles) d'acide 2-chloro-3-trifluorobenzoïque, on obtient, après traitement et purification par chromatographie sur gel de silice, 0,8 g d'érythro-2-chloro-*N*-(1-méthylazépan-2-yl)(phényl)méthyl]-3-(trifluorométhyl)benzamide.
On dissout ce dernier dans quelques ml de propan-2-ol, on ajoute 20 ml d'une solution 0,1N d'acide chlorhydrique dans le propan-2-ol, et on concentre le mélange sous pression réduite afin de réduire le volume du solvant. Après trituration on isole finalement 0,48 g de chlorhydrate sous forme d'un solide.
Point de fusion : 124-126°C.

Le tableau 1 qui suit illustre les structures chimiques et les points de fusion de quelques composés de l'invention. Dans la colonne "Sel", - désigne un composé à l'état de base, HCl désigne un chlorhydrate et tfa désigne un trifluoroacétate.
Le composé 7 existe sous forme de mélange d'érythro (7,5) et de thréo (2,5).

**Tableau 1**

| | | | | | | | |
|---|---|---|---|---|---|---|---|
| 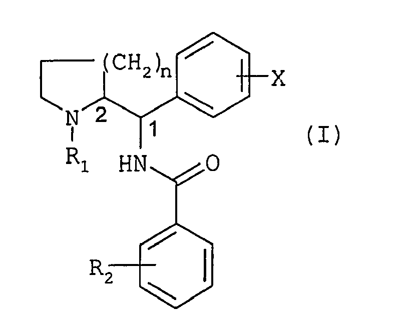 | | | | | | | |

| N° | stéréochimie | R₁ | n | X | R₂ | Sel | F (°C) |
|---|---|---|---|---|---|---|---|
| 1 | thréo (1*R*, 2*R*; 1*S*, 2*S*) | CH₃ | 1 | H | 2-Cl, 3-CF₃ | HCl | 96-110 |
| 2 | thréo (1*R*, 2*R*; 1*S*, 2*S*) | CH₃ | 1 | H | 2,6-Cl₂, 4-NH₂ | HCl | 155-162 |
| 3 | thréo (1*R*, 2*R*; 1*S*, 2*S*) | allyle | 1 | H | 2-Cl, 3-CF₃ | - | 117-123 |
| 4 | thréo (1*S*, 2*S*) | CH₃ | 1 | H | 4-Cl, 3-SO₂NH₂ | HCl | 165-170 |
| 5 | erythro (1*R*, 2*S*; 1*S*, 2*R*) | CH₃ | 1 | H | 3-Cl, 4-NH₂, 5-CF₃ | HCl | 130-140 |
| 6 | erythro (1*R*,2*S*) | CH₃ | 1 | H | 4-Cl, 3-SO₂NH₂ | HCl | 190-192 |
| 7 | erythro (1*R*, 2*S*; 1*S*, 2*R*) | CH₃ | 3 | H | 2-Cl, 3-CF₃ | HCl | 124-126 |

Les composés de l'invention ont été soumis à une série d'essais pharmacologiques qui ont mis en évidence leur intérêt comme substances à activités thérapeutiques.

### Etude du transport de la glycine dans les cellules SK-N-MC exprimant le transporteur humain glyt1 natif.

La capture de [¹⁴C]glycine est étudiée dans les cellules SK-N-MC (cellules neuro-épithéliales humaines) exprimant le transporteur humain glyt1 natif par la mesure de la radioactivité incorporée en présence ou en absence du composé à tester. Les cellules sont cultivées en monocouche pendant 48 h dans des plaques prétraitées à la fibronectine à 0,02%. Le jour de l'expérience, le milieu de culture est éliminé et les cellules sont lavées par un tampon Krebs-HEPES (acide [4-(2-hydroxyéthyl)pipérazine-1-éthanesulfonique) à pH 7,4. Après 10 min de préincubation à 37°C en présence soit de tampon (lot témoin), soit de composé à tester a différentes concentrations ou de 10 mM de glycine (détermination de la capture non spécifique), 10 µM de [¹⁴C] glycine (activité spécifique 112 mCi/mmole) sont ensuite ajoutés. L'incubation se poursuit pendant 10 min à 37°C, et la réaction est arrêtée par 2 lavages avec un tampon Krebs-HEPES à pH 7,4. La radioactivité incorporée par les cellules est alors estimée après ajout de 100 µl de scintillant liquide et agitation pendant 1 h. Le comptage est réalisé sur compteur Microbeta Tri-lux^{™}. L'efficacité du composé est déterminée par la CI₅₀, concentration du composé qui diminue de 50% la capture spécifique de glycine, définie par la différence de radioactivité incorporée par le lot témoin et le lot qui a reçu la glycine à 10 mM.
Les composés de l'invention, dans ce test, ont une CI₅₀ de l'ordre de 0,01 à 10 µM.

### Etude du transport de la glycine dans l'homogénat de moelle épinière de souris.

La capture de [¹⁴C] glycine par le transporteur glyt2 est étudiée dans l'homogénat de moelle épinière de souris par la mesure de radioactivité incorporée en présence ou en absence de composé à étudier.
Après euthanasie des animaux (souris mâles OF1 Iffa Crédo pesant 20 à 25 g le jour de l'expérience), la moelle épinière de chaque animal est rapidement prélevée, pesée et conservée sur glace. Les échantillons sont homogénéisés dans un tampon Krebs-HEPES (acide [4-(2-hydroxyéthyl)pipérazine-1-éthanesulfonique), pH 7,4, à raison de 25 ml/g de tissu.
50 µl d'homogénat sont pré-incubés pendant 10 min à 25°C en présence de tampon Krebs-HEPES , pH 7,4 et de composé à étudier à différentes concentrations, ou de 10 mM de glycine pour déterminer la capture non spécifique. La [¹⁴C]glycine (activité spécifique = 112mCi/mmole) est ensuite ajoutée pendant 10 min à 25°C à la concentration finale de 10 µM. La réaction est arrêtée par filtration sous vide et la radioactivité est estimée par scintillation solide par comptage sur un compteur Microbeta Tri-lux^{™}. L'efficacité du composé est déterminée par la concentration CI₅₀ capable de diminuer de 50% la capture spécifique de glycine, définie par la différence de radioactivité incorporée par le lot témoin et le lot qui a reçu la glycine 10 mM.
Les composés de l'invention dans ce test, ont une CI₅₀ de l'ordre de 0,1 à 10 µM.

Les résultats des essais effectués sur les composés de l'invention montrent qu'ils sont des inhibiteurs du transporteur de la glycine glytl présents dans le cerveau et glyt2 présents dans la moelle.

Ces résultats suggèrent que les composés de l'invention peuvent être utilisés pour le traitement des troubles comportementaux associés à la démence, des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.

Par ailleurs les composés de l'invention peuvent être utilisés pour le traitement des contractures musculaires douloureuses en rhumatologie et en pathologie rachidienne aiguë, pour le traitement des contractures spastiques d'origine médullaire ou cérébrale, pour le traitement symptomatique des douleurs aiguës et subaiguës d'intensité légère à modérée, pour le traitement des douleurs intenses et/ou chroniques, des douleurs neurogènes et algies rebelles, pour le traitement de la maladie de Parkinson et des symptômes parkinsoniens d'origine neurodégénérative ou induits par des neuroleptiques, pour le traitement des épilepsies généralisées primaires et secondaires, partielles à symptomatologie simple ou complexe, des formes mixtes et autres syndromes épileptiques en complément d'un autre traitement antiépileptique, ou en monothérapie, pour le traitement des apnées du sommeil, et pour la neuroprotection.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

### Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques (« patch »), des suppositoires. Pour l'administration topique on peut envisager des pommades, lotions et collyres.
Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de même qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylène glycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé répondant à la formule générale (I) 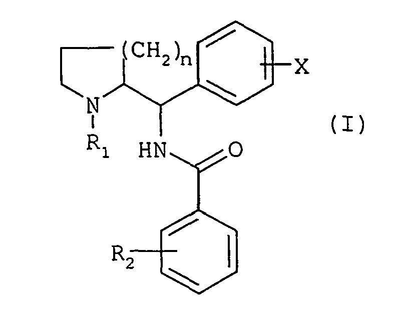 dans laquelle
n représente le nombre 1 ou 3,
R₁ représente soit un atome d'hydrogène, soit un groupe (C₁-C₇)alkyle linéaire ou ramifié éventuellement substitué par un ou plusieurs atomes de fluor, soit un groupe (C₃-C₇) cycloalkyle, soit un groupe (C₃-C₇)cycloalkyl-(C₁-C₃) alkyle, soit un groupe phényl (C₁-C₃) alkyle éventuellement substitué par un ou deux groupes méthoxy, soit un groupe (C₂-C₄)alcényle, soit un groupe (C₂-C₄) alcynyle,
X représente soit un atome d'hydrogène ou soit un ou plusieurs substituants choisis parmi les atomes d'halogènes et les groupes trifluorométhyle, (C₁-C₆)alkyles et (C₁-C₆)alcoxy linéaires ou ramifiés,
R₂ représente soit un atome d'hydrogène, soit un ou plusieurs substituants choisis parmi les atomes d'halogènes, et les groupes trifluorométhyle, (C₁-C₆)alkyles, (C₁-C₆) alcoxy, linéaires ou ramifiés, (C₃-C₇)cycloalkyles, phényle, cyano, acétyle, benzoyle, S(C₁-C₆)alkyles, (C₁-C₆) alkylsulfonyles, carboxy et (C₁-C₆)alcoxycarbonyles, soit un groupe de formule générale NR₃R₄, SO₂NR₃R₄ ou CONR₃R₄, dans lesquelles R₃ et R₄ représentent chacun, indépendamment l'un de l'autre, un atome d'hydrogène ou un groupe (C₁-C₆)alkyle linéaire ou ramifié ou (C₃-C₇)cycloalkyle, ou forment, avec l'atome d'azote qui les porte, un cycle pyrrolidine, pipéridine ou morpholine,
à l'état de base ou de sel d'addition à un acide.

2. Composé selon la revendication 1, **caractérisé en ce qu'**il est de configuration relative thréo (1*S*,2*S* ; *1R*, 2*R*).

3. Composé selon la revendication 1, **caractérisé en ce qu'**il est de configuration (1*S*,2*S*).

4. Composé selon la revendication 1, **caractérisé en ce qu'**il est de configuration (1*R*,2*R*).

5. Composé selon la revendication 1, **caractérisé en ce qu'**il est de configuration relative érythro (1*S*,2*R* ;1*R*, 2*S*).

6. Composé selon la revendication 1, **caractérisé en ce qu'**il est de configuration (1*R*,2*S*).

7. Composé selon la revendication 1, **caractérisé en ce qu'**il est de configuration (1*S*,2*R*).

8. Médicament, **caractérisé en ce qu'**il consiste en un composé selon l'une quelconque des revendications 1 à 5.

9. Composition pharmaceutique, **caractérisée en ce qu'**elle contient un composé selon l'une quelconque des revendications 1 à 5, associé à un excipient.

10. Utilisation d'un composé selon la revendication 1 pour la préparation d'un médicament destiné au traitement des troubles comportementaux associés à la démence, des psychoses, des diverses formes d'anxiété, des attaques de panique, des phobies, des troubles obsessionnels compulsifs, des différentes formes de dépression, des troubles dus à l'abus ou au sevrage d'alcool, des troubles du comportement sexuel, des troubles de la prise de nourriture et de la migraine.

11. Utilisation d'un composé selon la revendication 1 pour la préparation d'un médicament destiné au traitement des contractures, de la douleur, de la maladie de Parkinson et des symptômes parkinsoniens, des épilepsies, des formes mixtes et autres syndromes épileptiques en complément d'un autre traitement antiépileptique, ou en monothérapie, des apnées du sommeil, et pour la neuroprotection.

## Claims

1. Compound corresponding to the general formula (I) 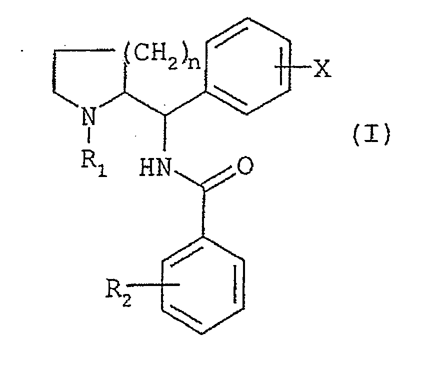 in which
n represents the number 1 or 3,
R₁ represents either a hydrogen atom, a linear or branched (C₁-C₇)alkyl group optionally substituted with one or more fluorine atoms, a (C₃-C₇)cycloalkyl group, a (C₃-C₇)cycloalkyl(C₁-C₃)alkyl group, a phenyl (C₁-C₃) alkyl group optionally substituted with one or two methoxy groups, a (C₂-C₄) alkenyl group, or a (C₂-C₄) alkynyl group,
X represents either a hydrogen atom or one or more substituents chosen from halogen atoms and trifluoromethyl and linear or branched (C₁-C₆) alkyl and (C₁-C₆) alkoxy groups,
R₂ represents either a hydrogen atom or one or more substituents chosen from halogen atoms and trifluoromethyl, linear or branched (C₁-C₆)alkyl and (C₁-C₆)alkoxy, (C₃-C₇)cycloalkyl, phenyl, cyano, acetyl, benzoyl, S(C₁-C₆)alkyl, (C₁-C₆)alkylsulfonyl, carboxyl and (C₁-C₆)alkoxycarbonyl groups, or a group of general formula NR₃R₄, SO₂NR₃R₄ or CONR₃R₄, in which R₃ and R₄ represent, independently of each other, a hydrogen atom or a linear or branched (C₁-C₆) alkyl or (C₃-C₇)cycloalkyl group, or form, with the nitrogen atom that bears them, a pyrrolidine, piperidine or morpholine ring,
in the form of base or of acid-addition salt.

2. Compound according to Claim 1, **characterized in that** it is of threo relative configuration (1*S*, 2*S*; 1*R*, 2*R*).

3. Compound according to Claim 1, **characterized in that** it is of (1*S*, 2*S*) configuration.

4. Compound according to Claim 1, **characterized in that** it is of (1*R*, 2*R*) configuration.

5. Compound according to Claim 1, **characterized in that** it is of erythro relative configuration (1*S*, 2*R*; 1*R*, 2*S*).

6. Compound according to Claim 1, **characterized in that** it is of (1*R*, 2*S*) configuration.

7. Compound according to Claim 1, **characterized in that** it is of (1*S*, 2*R*) configuration.

8. Medicament, **characterized in that** it consists of a compound according to any one of Claims 1 to 5.

9. Pharmaceutical composition, **characterized in that** it contains a compound according to any one of Claims 1 to 5, combined with an excipient.

10. Use of a compound according to Claim 1 for the preparation of a medicament for treating behavioral disorders associated with dementia, psychosis, various forms of anxiety, panic attacks, phobia, compulsive obsessive disorders, various forms of depression, disorders caused by alcohol abuse or weaning from alcohol, sexual behavior disorders, eating disorders and migraine.

11. Use of a compound according to Claim 1 for the preparation of a medicament for treating contracture, pain, Parkinson's disease and Parkinson-like symptoms, epilepsy, mixed forms and other epileptic syndromes in addition to another antiepileptic treatment, or in monotherapy, and sleep apnea, and for neuroprotection.

## Patentansprüche

1. Verbindung der allgemeinen Formel (I) 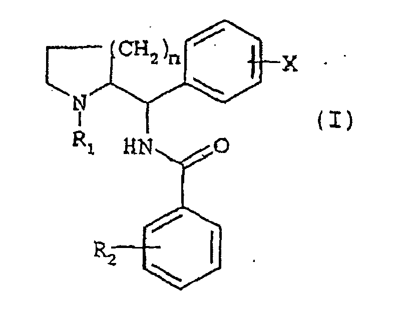 worin
n für die Zahl 1 oder 3 steht,
R₁ für ein Wasserstoffatom, eine gegebenenfalls durch ein oder mehrere Fluoratome substituierte lineare oder verzweigte (C₁-C₇)-Alkylgruppe, eine (C₃-C₇)-Cycloalkylgruppe, eine (C₃-C₇)-Cycloalkyl-(C₁-C₃)-alkylgruppe, eine gegebenenfalls durch eine oder zwei Methoxygruppen substituierte Phenyl-(C₁-C₃)-alkylgruppe, eine (C₂-C₄)-Alkenylgruppe oder eine (C₂-C₄)-Alkinylgruppe steht;
X für ein Wasserstoffatom oder einen oder mehrere unter Halogenatomen, Trifluormethyl- und linearen oder verzweigten (C₁-C₆)-Alkyl- und (C₁-C₆) - Alkoxygruppen ausgewählte Substituienten steht,
R₂ für ein Wasserstoffatom, einen oder mehrere unter Halogenatomen und Trifluormethyl-, linearen oder verzweigten (C₁-C₆)-Alkyl- und (C₁-C₆)-Alkoxy-, (C₃-C₇)-Cycloalkyl-, Phenyl-, Cyano-, Acetyl-, Benzoyl-, S- (C₁-C₆)-Alkyl-, (C₁-C₆)-Alkylsulfonyl-, Carboxy- und (C₁-C₆)-Alkoxycarbonylgruppen ausgewählte Substituienten oder eine Gruppe der allgemeinen Formel NR₃R₄, SO₂NR₃R₄ oder CONR₃R₄, worin R₃ und R₄ jeweils unabhängig voneinander ein Wasserstoffatom oder eine lineare oder verzweigte (C₁-C₆)-Alkyl- oder eine (C₃-C₇)-Cycloalkylgruppe bedeuten oder mit dem Stickstoffatom, an das sie gebunden sind, einen Pyrrolidin-, Piperidin- oder Morpholinring bilden, steht,
in Basen- oder Säureadditionssalzform.

2. Verbindung nach Anspruch 1, **dadurch gekennzeichnet, daß** sie in der threo-Relativkonfiguration (1*S*, 2*S*; 1*R*, 2*R*) vorliegt.

3. Verbindung nach Anspruch 1, **dadurch gekennzeichnet, daß** sie in der (1*S*,2*S*)-Konfiguration vorliegt.

4. Verbindung nach Anspruch 1, **dadurch gekennzeichnet, daß** sie in der (1*R*,2*R*)-Konfiguration vorliegt.

5. Verbindung nach Anspruch 1, **dadurch gekennzeichnet, daß** sie in der erythro-Relativkonfiguration (1*S*, 2*R*; 1*R*, 2*S*) vorliegt.

6. Verbindung nach Anspruch 1, **dadurch gekennzeichnet, daß** sie in der (1*R*,2*S*)-Konfiguration vorliegt.

7. Verbindung nach Anspruch 1, **dadurch gekennzeichnet, daß** sie in der (1*S*,2*R*)-Konfiguration vorliegt.

8. Medikament, **dadurch gekennzeichnet, daß** es aus einer Verbindung nach einem der Ansprüche 1 bis 5 besteht.

9. Pharmazeutische Zusammensetzung, **dadurch gekennzeichnet, daß** sie eine Verbindung nach einem der Ansprüche 1 bis 5 in Kombination mit einem Trägerstoff enthält.

10. Verwendung einer Verbindung nach Anspruch 1 zur Herstellung eines Medikaments zur Behandlung von Verhaltensstörungen in Assoziation mit Demenz, Psychosen, verschiedenen Formen von Angst, Panikattacken, Phobien, Zwangsneurosen, verschiedenen Formen von Depression, Störungen, die auf Alkoholmißbrauch oder -entzug zurückzuführen sind, Störungen des Sexualverhaltens, Störungen der Nahrungsaufnahme und Migräne.

11. Verwendung einer Verbindung nach Anspruch 1 zur Herstellung eines Medikaments zur Behandlung von Kontrakturen, Schmerz, Parkinson-Krankheit und Parkinson-ähnlichen Symptomen, Epilepsien, Mischformen und anderen epileptischen Syndromen zusätzlich zu einer anderen antiepileptischen Behandlung oder bei der Monotherapie und Schlafapnoe und zur Neuroprotektion.
